# EUROPEAN PATENT SPECIFICATION

(11) **EP 3 644 140 B1**
(45) Date of publication and mention of the grant of the patent: **07.07.2021**
(21) Application number: 19180258.6
(22) Date of filing: 14.06.2019
(51) Int. Cl.: G05B 15/02

(54) **SMART BUILDING SYSTEM**
INTELLIGENTES GEBÄUDESYSTEM
SYSTÈME DE CONSTRUCTION INTELLIGENT

(30) Priority: 23.10.2018 KR 20180126431
(43) Date of publication of application: 29.04.2020
(73) Proprietor: Xandar Kardian, Seoul 04793 (KR)
(72) Inventor: YANG, Sun Jong, 26101 Bukpyeong-myeon, Jeongseon-gun, Gangwon-do (KR); CHOI, Jeong Woo, 04715 Seoul (KR)
(74) Representative: Viering, Jentschura & Partner mbB Patent- und Rechtsanwälte

(56) References cited:
- US-A1- 2007 173 978
- US-A1- 2015 156 031
- US-A1- 2017 093 594
- US-A1- 2018 077 778

## Description

### BACKGROUND OF THE INVENTION

### Field of the Invention

The present invention relates to a smart building system that senses a person located in an indoor space to automatically control an indoor controller disposed in the indoor space.

### Description of the Related Art

With the development of science and technology, there is an increase in demand for efficient management of office buildings and accommodations such as hotels. A power saving type sensing apparatus has been widely used that senses a human body or the like and operates the illuminating device by reflecting the sensing results for the purpose of efficient management. A sensor capable of sensing the heat and motion of a human body in the related art is configured in such a manner as to be applied to identify or sense a case where a movement of a person occurs. That is, when a person enters within the sensing range of the sensor, the illuminating device is activated by sensing the presence of the person, but the sensor does not sense daily movements in the indoor space. In addition, the sensors in the related art have limitations that they are used only for turning on/off a illuminating device.

In addition, since a person is present in an indoor space of office buildings and accommodations, an administrator cannot enter the indoor space or control the indoor controller disposed in the indoor space without permission. Accordingly, there is a problem that the illuminating device is turned on or the air conditioner is turned on even though there is no person in the indoor space thereby causing power waste.

From US 2007/173978 A1, US 2015/156031 A1, US 2018/077778 A1 and US 2017/093594 A1 smart building systems according to the preamble of claim 1 are known.

### SUMMARY OF THE INVENTION

The present invention has been made keeping in mind the above problems occurring in the related art, and an object of the present invention is to provide a smart building system that senses a person located in an indoor space to automatically control an indoor controller disposed in the indoor space.

It is an object of the present invention to provide a smart building system that determines in which space of an indoor space a person is located to control the driving strength of an indoor controller in a certain space of the indoor space.

In order to accomplish the above object, the present invention provides a smart building system according to claim 1. Preferable embodiments are subject of the dependent claims. The smart building system includes a sensor unit disposed in an indoor space to sense a presence of a person located in the indoor space and biometric information of the person located in the indoor space, a determination unit determining whether or not the person is located in the indoor space on the basis of the information acquired by the sensor unit, and a control unit controlling an indoor controller disposed inside the indoor space on the basis of the information determined by the determination unit.

According to an example, the determination unit may adjust a sensitivity of the sensor unit according to a driving strength of the indoor controller.

According to an example, the indoor controller may be an air conditioner, in which an air flow becomes strong in the indoor space as a driving strength of the air conditioner becomes strong, and the determination unit reduces a sensitivity of the sensor unit in order to prevent a sensing error of the sensor unit due to the strong change in the air flow.

According to an example, the determination unit may include an analysis unit analyzing whether or not a movement sensed in the indoor space is generated by the person on the basis of the information sensed by the sensor unit, a sensitivity adjustment unit controlling a sensitivity of the sensor unit on the basis of a driving strength of the indoor controller, and a storage unit storing information about repetitive movements that occur in the indoor space.

The analysis unit determines that the same information as the information about the repetitive movements stored in the storage unit among the information sensed by the sensor unit is not the biometric information of the person.

According to an example, the smart building system may further include a big data generation unit storing information about a behavior pattern of the person located in the indoor space, a driving pattern of the indoor controller, and the indoor space, thereby collecting information about a specific person.

According to an example, the information about the specific person generated by the big data generation unit may be associated with a reservation system of an accommodation that is occupied by the specific person, and the information stored in the big data generation unit may be transmitted to the reservation system of the accommodation when the specific person reserves the accommodation.

According to an example, the determination unit may determine the number of persons who are located in the indoor space, and the control unit may control a strength of the indoor controller on the basis of the number of persons.

According to an example, the determination unit may transmit the determined information to a terminal disposed outside the indoor space.

According to an example, the terminal may be attached to the outside of the indoor space to display whether or not the person is located inside the indoor space.

According to an example, the determination unit may be connected to the control unit via a server.

The biometric information includes at least one of heart rate, movement, or respiration of the person.

According to an example, the determination unit may analyze a behavior pattern and a sleeping pattern of the person present in the indoor space on the basis of the biometric information sensed by the sensor unit.

The sensor unit includes an impulse-radio ultra-wideband (IR-UWB) communication sensor.

According to an example, the sensor unit may be provided in multiple in the indoor space, the multiple sensor units may sense whether or not the person is present in a private space or a public space among the indoor spaces, and the indoor controller may be provided in each of the private space and the public space.

According to an example, the determination unit may determine whether or not the person is present in the private space and the public space on the basis of the information sensed by the multiple sensor units, and the control unit may control the indoor controller disposed in a space where no person is present among the private space and the public space.

According to an example, the indoor controller may include an air conditioner and an illuminating unit, and the control unit may control a driving strength of the air conditioner disposed in the space where the person is not present among the private space and the public space to be lower than a current driving strength and an intensity of illumination of the illuminating unit to be lower than a current intensity.

According to an example, the indoor controller may include an air conditioner and an illuminating unit, and when a person located in any one space of the private space and the public space moves to the other space, the control unit may control a driving strength of the air conditioner to be lower than a current strength and an intensity of illumination of the illuminating unit to be lower than a current intensity in the air conditioner and the illuminating unit disposed in the any one space.

According to an example, when a person located in any one space of the private space and the public space moves to the other space, the control unit may control the driving strength of the air conditioner to be higher than the current strength and the intensity of illumination of the illuminating unit to be higher than the current intensity in the air conditioner and the illuminating unit disposed in the other space.

According to an embodiment of the present invention, since the smart building system can control the indoor controller on the basis of whether or not there is a person in the indoor space, it is possible to prevent the indoor controller from being turned on and thus power from being unnecessarily wasted even though there is no person in the indoor space.

According to an embodiment of the present invention, the smart building system stores, in advance, information on a behavior pattern of a person located in an indoor space, a driving pattern of an indoor controller, a sleeping pattern of a person, and the like, so that the indoor space can be properly set for the person or the administrator can be informed of the proper setting state of the indoor space.

Since the smart building system according to an embodiment of the present invention can acquire information about a person through the radar, it is possible to determine whether a person is located in the indoor space, in which space of the indoor space the person is located, and the number of persons who are located in the indoor space. In addition, the smart building system can adjust the driving strength of the indoor controller on the basis of the result of sensing the indoor space, thereby preventing unnecessary power waste.

### BRIEF DESCRIPTION OF THE DRAWINGS

The above and other objects, features and other advantages of the present invention will be more clearly understood from the following detailed description when taken in conjunction with the accompanying drawings, in which:
FIG. 1 is a block diagram illustrating a smart building system according to an embodiment of the present invention;
FIG. 2 is a view illustrating an indoor space in which a smart building system is implemented according to an embodiment of the present invention;
FIG. 3 is a block diagram illustrating a determination unit of FIG. 1;
FIG. 4 is a block diagram illustrating a terminal controlled by a smart building system according to an embodiment of the present invention;
FIG. 5 is a diagram illustrating how a smart building system is associated with a reservation system of accommodation according to an embodiment of the present invention;
FIG. 6 is a diagram illustrating a private space and a public space in which a smart building system is implemented according to an embodiment of the present invention; and
FIG. 7 is a block diagram illustrating a smart building system according to another embodiment of the present invention.

### DETAILED DESCRIPTION OF THE INVENTION

The advantages and features of the present invention and the manner of achieving them will become apparent with reference to the embodiments described in detail below with reference to the accompanying drawings. The present invention may, however, be embodied in many different forms and should not be construed as limited to the embodiments set forth herein. Rather, these embodiments are provided so that this disclosure will be thorough and complete, and will fully convey the concept of the invention to those skilled in the art. Furthermore, the present invention is defined only by the scope of claims. Like reference numerals refer to like elements throughout the specification.

In addition, the embodiments described herein will be described with reference to cross-sectional views and/or plan views, which are ideal illustrations of the present invention. In the drawings, the thicknesses of the films and regions are exaggerated for an effective explanation of the technical content. Thus, the shape of the illustrations may be modified by manufacturing techniques and/or tolerances. Accordingly, the embodiments of the present invention are not limited to the specific shapes shown, but also include changes in shapes that are produced according to the manufacturing process. For example, the etching regions shown at right angles may be rounded or may have a shape with a certain curvature. Thus, the regions illustrated in the figures have schematic attributes, and the shapes of the regions illustrated in the figures are intended to illustrate specific types of regions of the elements and are not intended to limit the scope of the invention.

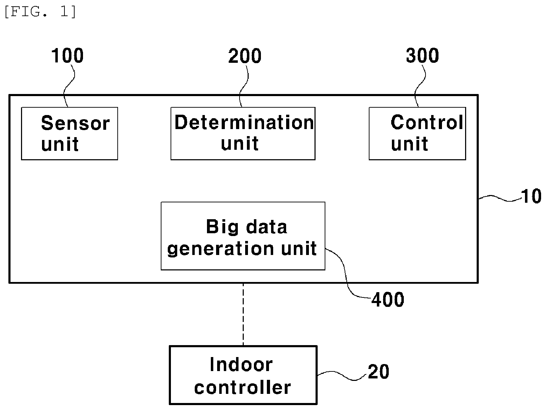

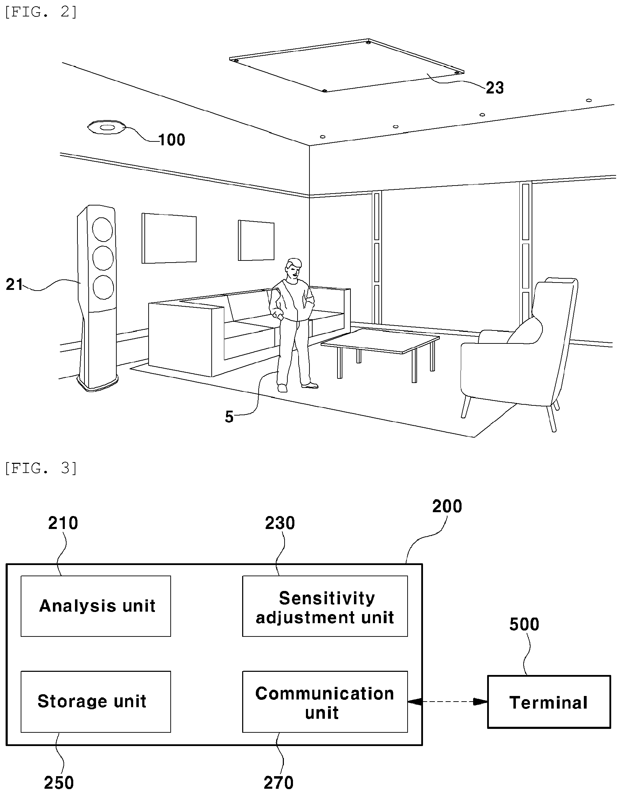

FIG. 1 is a block diagram illustrating a smart building system according to an embodiment of the present invention, and FIG. 2 is a view illustrating an indoor space in which a smart building system is implemented according to an embodiment of the present invention.

Referring to FIGS. 1 and 2, a smart building system 10 may include a sensor unit 100, a determination unit 200, a control unit 300, and a big data generation unit 400. The smart building system 10 may communicate wirelessly or by wire with an indoor controller 20 disposed in an indoor space where a person 5 is located. The smart building system 10 may be applied to office buildings or accommodations such as hotels. The sensor unit 100, the determination unit 200, the control unit 300, and the big data generation unit 400 according to an embodiment of the present invention may be configured in such a manner as to be included in one device, and only partial configurations of the sensor unit 100, determination unit 200, the control unit 300, and the big data generation unit 400 may be included in one device. For example, the sensor unit 100, the determination unit 200, and the control unit 300 may be included in one device disposed in the indoor space. The sensor unit 100, the determination unit 200, the control unit 300, and the big data generation unit 400 may be combined and provided in various ways to implement the smart building system 10.

The sensor unit 100 may be installed in an indoor space to sense the presence of the person 5 who is located in the indoor space and the biometrical information of the person 5 located in the indoor space. The sensor unit 100 may be attached to a ceiling or a wall surface in the indoor space, but the position where the sensor unit 100 is attached may not be particularly limited. The sensor unit 100 may sense objects moving within the indoor space. Therefore, the sensor unit 100 may sense the person 5 moving in the indoor space, and may sense the biometric information of the person 5 thereby sensing a person 5 who is asleep. The sensor unit 100 may sense a minute movement generated by the object according to changes in the driving strength of the indoor controller 20 located in the indoor space. For example, the indoor controller 20 may be an air conditioner 21 and an illuminating unit 23, in which the air flow in the indoor space may become stronger as the driving strength of the air conditioner 21 becomes stronger so that the sensor unit 100 may sense objects that move minutely due to the strong air flow. For example, the air conditioner 21 may include an air conditioner, a heater, a humidifier, an air purifier, and the like.

The sensor unit 100 may be any one of an impulse-radio ultra wideband communication (IR-UWB) sensor, a Lidar, a frequency modulated continuous wave (FMCW) radar, and a Doppler radar. Preferably, the sensor unit 100 may be an IR-UWB communication sensor. The UWB communication refers to radio technology that uses a frequency band of 500 MHz or more, or is defined as a signal having a fractional bandwidth of at least 25%. The fractional bandwidth means the bandwidth of the signal compared to its center frequency. The UWB communication is a radio technology that uses broadband frequencies, and has various advantages such as high range resolution, transmittance, strong immunity to narrowband noise, and coexistence with other devices sharing frequency. For example, the UWB has the advantage of sensing minute movements of an object because of ultra-precise distance resolution characteristics of 1 cm or less.

An impulse-radio ultra wideband radar (hereinafter, referred to as, "UWB radar") technology is a system in which the UWB communication technology is combined with radar and refers to a radar technology that transmits a very short duration-impulse signal having a wideband characteristic in a frequency domain and receives a signal reflected from objects and person 5 thereby recognizing the surrounding situation. The UWB radar system generates an impulse signal with a time width of several nanoseconds to several picoseconds in the signal generator and emits it at a wide angle or narrow band angle through a transmitting antenna. The emitted signal is reflected by various objects or person 5 in the environment, and the reflected signal may be converted to a digital signal through a receiving antenna and an analog-to-digital converter (ADC) .

The sensor unit 100 may sense the biometric information of the person 5 located in the indoor space. The biometric information may include at least one of the heart rate, movement, or respiration of the person 5. The sensor unit 100 receives the signal reflected by the person 5 and may sense the movement of the chest or abdomen of the person 5, whereby it is possible to sense the heart rate or respiration of the person 5. Also, the sensor unit 100 may receive the reflected signal in real time and sense the movement of the person 5. Also, the sensor unit 100 may sense the number of persons 5 located in the indoor space.

The determination unit 200 may determine whether or not the person 5 is located in the indoor space on the basis of the information acquired by the sensor unit 100. The determination unit 200 may analyze the information acquired by the sensor unit 100 to determine whether the sensed movement is a movement of an object or a movement of a person 5. The determination unit 200 may adjust the sensitivity of the sensor unit 100 when the sensed movement is a minute movement that is not generally regarded as the movement of the person 5. That is, the determination unit 200 may lower the sensitivity of the sensor unit 100 to prevent the sensor unit 100 from sensing movements other than the movement of the person 5. In addition, the determination unit 200 may lower the sensitivity of the sensor unit 100 as the driving strength of the indoor controller 20 is increased. When the driving strength of the air conditioner 21 in the indoor controller 20 is increased, an object located in the vicinity of the air conditioner 21 may be moved by the wind generated by the air conditioner 21. For example, when the air conditioner is strongly driven, minute movements may occur in plants, curtains, book pages, and the like located in the vicinity of the air conditioner, so that the sensor unit 100 may sense such minute movements of the plants, curtains, and book pages. Therefore, when the determination unit 200 determines that the information acquired by the sensor unit 100 is a movement of an object located in the vicinity of the air conditioner 21 due to the wind generated by the air conditioner 21, it is possible to lower the sensitivity of the sensor unit 100. As the sensitivity of the sensor unit 100 is lowered, the minute movement caused by the wind generated in the air conditioner 21 is not sensed by the sensor unit 100. Therefore, it is possible to increase the probability that the sensor unit 100 senses only the movement of the person 5.

The determination unit 200 may analyze multiple movements sensed by the sensor unit 100 and determine how many persons 5 are located in the indoor space. The determination unit 200 may transmit the analyzed information to a terminal located outside the indoor space.

The determination unit 200 may analyze a behavior pattern and a sleeping pattern of a person 5 present in the indoor space on the basis of the biometric information sensed by the sensor unit 100. For example, the sensor unit 100 may sense how much time the person 5 spends in a particular space of the indoor space, so that the determination unit 200 may analyze the behavior pattern of the person 5 in the indoor space on the basis of the information sensed by the sensor unit 100. For example, the sensor unit 100 senses a sleeping pattern including the sleeping time, the quality of the sleeping, and how much the person 5 is moving during the sleeping through the biometric information of the person 5 when the person 5 is in sleep, and the determination unit 200 may analyze the sleeping pattern of a person on the basis of information sensed by the sensor unit 100. Herein, the quality of the sleeping may mean information including whether the person 5 is constantly breathing during sleeping, and whether there are any symptoms of sleep apnea, and so on.

The control unit 300 may control the indoor controller 20 disposed in the indoor space on the basis of the information determined by the determination unit 200. For example, when the determination unit 200 determines that the person 5 does not exist in the indoor space, the control unit 300 may turn off the operation of the indoor controller 20. In addition, when the determination unit 200 determines that the person 5 does not exist in the indoor space and then the movement of the person 5 is sensed, the control unit 300 may turn on the operation of the indoor controller 20.

When the determination unit 200 determines how many persons 5 are located in the indoor space, the control unit 300 may control the driving strength of the indoor controller 20 on the basis of the number of people 5. For example, the control unit 300 may increase the driving strength of the air conditioner 21 as the number of persons 5 located in the indoor space increases. On the contrary, the control unit 300 may reduce the driving strength of the air conditioner 21 as the number of persons 5 located in the indoor space decreases. The driving strength of the air conditioner 21 is controlled according to the number of the persons 5 located in the indoor space, so that it is possible to prevent the phenomenon that the air conditioner 21 is driven uselessly and thus power is wasted.

The big data generation unit 400 stores information about a behavior pattern of a person located in an indoor space, a driving pattern of the indoor controller 20, and the indoor space, thereby collecting information about the person 5 located in a specific indoor space. The information collected by the big data generation unit 400 includes predetermined information such as information on the indoor space and information such as behavior patterns that may be varied according to the person 5 located in the indoor space and driving pattern of the indoor controller 20. In a case that the smart building system 10 is applied to a hotel, when a previous occupant person 5 stays at the hotel again, the smart building system 10 may set the setting of the indoor space, the basic driving strength of the indoor controller 20, and the like on the basis of the information collected by the big data generation unit 400. For example, the smart building system 10 controls the indoor controller 20 in the same manner as the driving strength of the indoor controller 20 set by the person 5 when the person 5 has stayed at the hotel before, thereby maintaining the indoor space at a proper temperature in advance.

The smart building system 10 according to an embodiment of the present invention may control the indoor controller 20 on the basis of whether or not the person 5 is present in the indoor space, whereby it is possible to prevent the indoor controller 20 from being turned on and thus power from being unnecessarily wasted even though there is no person 5 in the indoor space. The smart building system 10 stores information about the behavior pattern of the person 5 located in the indoor space, the driving pattern of the indoor controller 20, the sleeping pattern of the person 5, and the like, whereby it is possible to set the indoor space in a proper state for the person 5 in the state or inform an administrator of the proper setting state for the indoor space.

Since the smart building system 10 according to an embodiment of the present invention may acquire information about the person 5 through the radar, it is possible to determine accurately the presence of the person 5 who is located in the indoor space and the number of persons 5 located in the indoor space. In addition, the smart building system 10 may adjust the driving strength of the indoor controller 20 on the basis of the result of sensing the indoor space, thereby preventing unnecessary power wastage.

FIG. 3 is a block diagram illustrating a determination unit of FIG. 1.

Referring to FIGS. 1 and 3, the determination unit 200 may include an analysis unit 210, a sensitivity adjustment unit 230, a storage unit 250, and a communication unit 270. The determination unit 200 may exchange information with the terminal 500 located outside the indoor space through the communication unit 270. The terminal 500 may be used in a central control system of a building or may be used as a display device positioned adjacent to a door for accessing to and exiting from the indoor space to display the state of the indoor space. However, a type of the terminal 500 may not be limited to the above.

The analysis unit 210 may analyze whether or not the motion sensed in the indoor space is generated by the person 5 on the basis of the information sensed by the sensor unit 100. The analysis unit 210 may determine that when the movements sensed in the indoor space are repetitive at a predetermined cycle, that movements are not the movements of the person 5. In addition, the analysis unit 210 may determine that minute or fast motion that is not determined to be the movement of the person 5 is not the movement of the person 5. For example, repetitive movements that move at regular intervals may include rotations of the fan, movements of a second hand of a clock, and the like. For example, the movements of plant leaves and the fluttering of the book pages that are rapidly trembled due to the wind produced by an air conditioner and may be determined not to be the movements of the person 5 by the analysis unit 210.

The sensitivity adjustment unit 230 may control the sensitivity of the sensor unit 100 in consideration of the driving strength of the indoor controller 20 and the measurement frequency of movement other than the movement of the person 5 sensed by the sensor unit. For example, when the indoor controller 20 such as an air conditioner is strongly driven, the sensor unit 100 continuously senses the movements of minute and fast objects, whereby the sensitivity adjustment unit 230 may lower the sensitivity of the sensor unit 100. As the sensitivity adjustment unit 230 adjusts the sensitivity of the sensor unit 100, only the movements of the person 5 are sensed by the sensor unit 100, so that the determination unit 200 may accurately determine whether or not the person 5 is located in the indoor space.

The storage unit 250 may store information about repetitive movements that may occur in the indoor space. The repetitive movements may mean moving with a constant cycle. For example, the repetitive movements may include rotations of a fan, movement of a second hand of a clock, and the like. In addition, the storage unit 250 may store information about minute or fast movements, etc., which are not determined to be the movements of the person 5. The same determination unit 200 may determined that repetitive movements stored in the storage unit 250 and information that is not determined to be the movement of the person 5 among the movements sensed by the sensor unit 100 are not the movement of the person 5. Accordingly, the determination unit 200 may analyze whether or not the person 5 resides in the indoor space only using the movement of the person 5 among various information measured by the sensor unit 100.

The communication unit 270 may transmit information about whether or not the person 5 exists in the indoor space determined by the analysis unit 210 to the terminal 500 outside the indoor space.

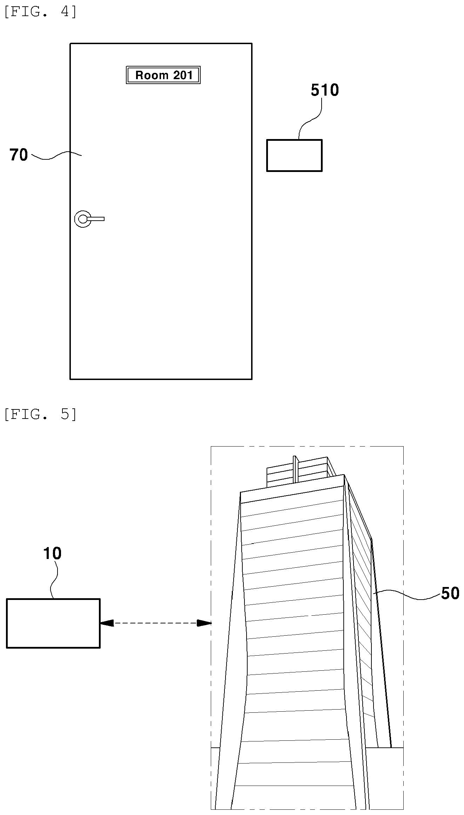

FIG. 4 is a block diagram illustrating a terminal controlled by a smart building system according to an embodiment of the present invention.

Referring to FIGS. 1, 3, and 4, the terminal 500 may be a display device 510 attached to the outside of the indoor space to indicate whether or not a person resides in the indoor space. For example, the display device 510 may be disposed adjacent to a door 70 for entering the interior space. The display device 510 may indicate whether or not a person is present inside the indoor space, thereby allowing persons outside the indoor space to know that. For example, when the smart building system 10 is used in an office building, the display device 510 may display whether there is a person currently located inside the office. For example, when the smart building system 10 is used in an accommodation, the display device 510 displays whether or not a person is located in the indoor space so that an accommodation staff who cleans the indoor space may freely enter the indoor space.

The smart building system 10 according to an embodiment of the present invention displays whether or not a person is located in the indoor space in order to allow the persons outside the indoor space to know that, whereby the staff who cleans the indoor space does not need to separately determine whether not the person is located inside the indoor space. Generally, since the staff who cleans the indoor space may not clearly know whether or not a guest is located in the indoor space, the indoor space may not be cleaned until a predetermined time even if the guest occupied at the accommodation goes out early in the morning. However, the smart building system 10 according to an embodiment of the present invention senses the presence of the guest in the indoor space and displays the information, so that the staff who cleans the indoor space may know that there is no guest in the indoor space through the display device 510 and clean the indoor space even before the predetermined time. Therefore, it is possible to efficiently use a time determined to clean a plurality of indoor spaces.

FIG. 5 is a diagram illustrating how a smart building system is associated with a reservation system of accommodation according to an embodiment of the present invention.

Referring to FIGS. 1 and 5, information sensed and prestored by the smart building system 10 may be shared with a reservation system 50 of an accommodation. That is, the information about the specific person using the specific indoor space generated by the big data generation unit 400 may be associated with the reservation system 50 of the accommodation. Information about the behavior pattern of the person, the sleeping pattern, and the driving pattern of the indoor controller 20 used by the person in the indoor space is collected by the big data generation unit 400, and the collected information is transmitted to the reservation system 50 of the accommodation. When the same person reserves the accommodation, the reservation system 50 may know information about the intensity of illumination of the indoor space, the temperature, and the things heavily used by persons in the indoor space on the basis of the previously stored information. For example, since the reservation system 50 has information on the sleeping pattern of a person, it is possible to know in advance information about bedding or the like used when a person's sleeping quality is high and let an administrator set the proper bedding and the like in the indoor space. For example, when a person is located in the indoor space, the reservation system 50 may know information about the intensity of illumination of the illuminating unit, the room temperature, and the like in advance, thereby allowing the smart building system 10 to automatically set the intensity of illumination and the temperature of the indoor space.

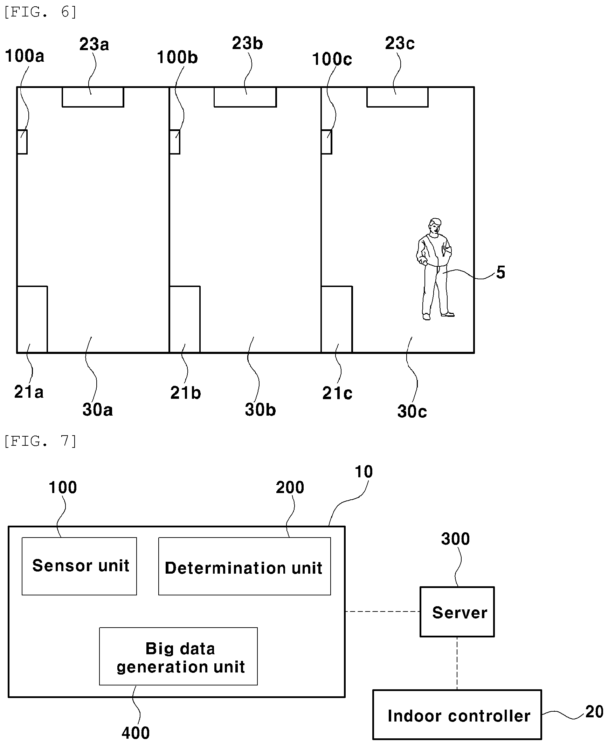

FIG. 6 is a diagram for illustrating a private space and a public space in which a smart building system is implemented according to an embodiment of the present invention.

Referring to FIGS. 1 and 6, the indoor space may be divided into a plurality of spaces. For example, the indoor space may be divided into a private space and a public space. The indoor controller 20 may be provided in each of the private space and the public space. In the present embodiment, the indoor space is divided into a first space 30a, a second space 30b, and a third space 30c, and each of the first space 30a, the second space 30b, and the third space 30c may be the private space or the public space. Air conditioners 21a, 21b, and 21c, illumination units 23a, 23b, and 23c, and sensor units 100a, 100b, and 100c may be provided in the first space 30a, the second space 30b, and the third space 30c, respectively. The first air conditioner 21a, the first illumination unit 23a, and the first sensor unit 100a may be disposed in the first space 30a, and the second air conditioner 21b, the second illumination unit 23b, and the second sensor unit 100b may be disposed in the second space 30b. The third air conditioner 21c, the third illumination unit 23c, and the third sensor unit 100c may be disposed in the third space 30c.

The determination unit 200 may determine in which space of the indoor space the person 5 is present on the basis of information sensed by the plurality of sensor units 100a, 100b, and 100c. The control unit 300 may control the air conditioners 21a, 21b, and 21c and the illuminating units 23a, 23b, and 23c disposed in the indoor space where the person 5 is not present.

For example, the determination unit 200 may sense the presence of the person 5 located in the third space 30c. Then, the control unit 300 may control the driving strength of each of the first air conditioner 21a and the second air conditioner 21b located in the first space 30a and the second space 30b to be lower than the current driving strength and the intensity of illumination of each of the first illuminating unit 23a and the second illuminating unit 23b to be lower than the current intensity.

As another example, when the person 5 located in the third space 30c moves to the second space 30b, the determination unit 200 may determine that the person 5 has moved into the second space 30b. The control unit 300 may control the driving strength of the third air conditioner 21c located in the third space 30c to be lower than the current driving strength and the intensity of illumination of the third illuminating unit 23c to be lower than the current intensity. Also, the control unit 300 may control the driving strength of the second air conditioner 21b located in the second space 30b to be higher than the current driving force, and the intensity of illumination of the second illuminating unit 23b to be higher than the current intensity. That is, the determination unit 200 may determine which space of the indoor space the person 5 is located in, and the control unit 300 may control the operation of the indoor controller 20 located in a specific space among the plurality of spaces on the basis of which space the person 5 is located in.

FIG. 7 is a block diagram illustrating a smart building system according to another embodiment of the present invention.

Referring to FIG. 7, a smart building system 10 may include a sensor unit 100, a determination unit 200, and a big data server 400. The smart building system 10 may be operated in conjunction with a server 300 and may control an indoor controller 20 through the server 300.

The server 300 may be a central control system of a building to which the smart building system 10 is applied and may control the indoor controller 20 disposed in the indoor space on the basis of information obtained by the smart building system 10. The indoor controller 20 may be automatically controlled by the server 300 according to a predetermined set value and the indoor controller 20 may be controlled manually by an administrator managing the central control system through the server 300. That is, a type of control unit for controlling the indoor controller 20 may be a configuration of the server 300, and the determination unit 200 and the control unit (not shown) of the smart building system 10 may be connected through the server 300.

While the present invention has been described in connection with accompanying drawings, it will be understood by those skilled in the art that the present invention may be embodied in other specific forms without departing from the essential characteristics thereof as defined in claim 1. Therefore, it will be understood that the above-described embodiments are illustrative in all aspects and not restrictive.

## Claims

1. A smart building system (10), comprising:
a sensor unit (100) disposed in an indoor space to sense information which comprising a presence of a person (5) located in the indoor space and biometric information of the person (5) located in the indoor space;
a determination unit (200) determining whether or not the person (5) is located in the indoor space on the basis of the information acquired by the sensor unit (100);
a control unit (300) controlling an indoor controller (20) disposed inside the indoor space on the basis of the information determined by the determination unit (200); and
a storage unit (250),
**characterized in that**
the storage unit (250) is storing information about repetitive movements that occur in the indoor space,
wherein the determination unit (200) determines that information being the same as the information about the repetitive movements stored in the storage unit (250) among the information sensed by the sensor unit (100) is not the biometric information of the person (5) so as to determine whether or not the person (5) is located in the indoor space on the basis of the biometric information of the person (5),
the repetitive movements mean moving with a constant cycle,
the sensor unit (100) includes an impulse-radio ultra-wideband (IR-UWB) communication sensor,
the sensor unit (100) senses biometric information of the person (5) by transmitting a signal and receiving the signal reflected by the person (5), and
the biometric information includes at least one of heart rate, movement, or respiration of the person.

2. The system (10) of claim 1, wherein the determination unit (200) adjusts a sensitivity of the sensor unit (100) according to a driving strength of the indoor controller (20).

3. The system (10) of claim 1 or 2, wherein the indoor controller (20) is an air conditioner (21), in which an air flow becomes strong in the indoor space as a driving strength of the air conditioner (21) becomes strong, and the determination unit (200) reduces a sensitivity of the sensor unit (100) in order to prevent a sensing error of the sensor unit due to the strong change in the air flow.

4. The system (10) of any one of claims 1 to 3, wherein the determination unit (200) includes:
an analysis unit (210) analyzing whether or not a movement sensed in the indoor space is generated by the person (5) on the basis of the information sensed by the sensor unit (100); and
a sensitivity adjustment unit (230) controlling a sensitivity of the sensor unit (100) on the basis of a driving strength of the indoor controller (20).

5. The system (10) of any one of claims 1 to 4, further comprising:
a big data generation unit (400) storing information about a behavior pattern of the person (5) located in the indoor space, a driving pattern of the indoor controller (20), and the indoor space, thereby collecting information about a specific person (5).

6. The system (10) of claim 5, wherein the information about the specific person (5) generated by the big data generation unit (400) is associated with a reservation system (50) of an accommodation that is occupied by the specific person (5), and
the information stored in the big data generation unit (400) is transmitted to the reservation system (50) of the accommodation when the specific person (5) reserves the accommodation.

7. The system (10) of any one of claims 1 to 6, wherein the determination unit (200) determines the number of persons (5) who are located in the indoor space, and
the control unit controls a strength of the indoor controller (20) on the basis of the number of persons (5).

8. The system (10) of any one of claim 1, wherein the determination unit (200) transmits the determined information to a terminal (500) disposed outside the indoor space.

9. The system (10) of any one of claims 1 to 8, wherein the sensor unit (100) is provided in multiple in the indoor space,
the multiple sensor units (100) sense whether or not the person (5) is present in a private space or a public space among the indoor spaces, and
the indoor controller (20) is provided in each of the private space and the public space.

10. The system of claim 9, wherein the determination unit (200) determines whether or not the person (5) is present in the private space and the public space on the basis of the information sensed by the multiple sensor units (100), and
the control unit (300) controls the indoor controller (20) disposed in a space where no person (5) is present among the private space and the public space.

11. The system (10) of claim 9 or 10, wherein the indoor controller (20) includes an air conditioner (21) and an illuminating unit (23), and
the control unit (300) controls a driving strength of the air conditioner (21) disposed in the space where the person (5) is not present among the private space and the public space to be lower than a current driving strength and an intensity of illumination of the illuminating unit (23) to be lower than a current intensity.

12. The system (10) of claim 9 or 10, wherein the indoor controller (20) includes an air conditioner (21) and an illuminating unit (23), and
when a person located (5) in any one space of the private space and the public space moves to the other space, the control unit (300) controls a driving strength of the air conditioner (21) to be lower than a current strength and an intensity of illumination of the illuminating unit (23) to be lower than a current intensity in the air conditioner (21) and the illuminating unit (23) disposed in the any one space.

13. The system (10) of claim 12, wherein when a person (5) located in any one space of the private space and the public space moves to the other space, the control unit (300) controls the driving strength of the air conditioner (21) to be higher than the current strength and the intensity of illumination of the illuminating unit (23) to be higher than the current intensity in the air conditioner (21) and the illuminating unit (23) disposed in the other space.

## Patentansprüche

1. Intelligentes Gebäudesystem (10), welches aufweist:
eine Sensor-Einheit (100), welche in einem Innenraum angeordnet ist, um Informationen zu detektieren, welche aufweisen eine Anwesenheit einer Person (5), welche sich in dem Innenraum befindet, und biometrische Informationen der Person (5), welche sich in dem Innenraum befindet,
eine Ermittlungseinheit (200), welche ermittelt, ob sich die Person (5) in dem Innenraum befindet oder nicht, auf der Basis der Informationen, welche mittels der Sensor-Einheit (100) erfasst werden,
eine Steuereinheit (300), welche eine Innenraum-Steuervorrichtung (20) steuert, welche innerhalb des Innenraums angeordnet ist, auf der Basis der Informationen, welche mittels der Ermittlungseinheit (200) ermittelt werden, und
eine Speicher-Einheit (250),
**dadurch gekennzeichnet, dass**
die Speicher-Einheit (250) Informationen über sich wiederholende Bewegungen speichert, welche in dem Innenraum auftreten,
wobei die Ermittlungseinheit (200) ermittelt, dass Informationen, welche dieselben sind wie die Informationen über die sich wiederholenden Bewegungen, welche in der Speicher-Einheit (250) gespeichert sind, von den Informationen, welche mittels der Sensor-Einheit (100) detektiert werden, nicht die biometrischen Informationen der Person (5) sind, um zu ermitteln, ob sich die Person (5) in dem Innenraum befindet oder nicht, auf der Basis der biometrischen Informationen der Person (5),
die sich wiederholenden Bewegungen ein Bewegen mit einem konstanten Zyklus bedeuten,
die Sensor-Einheit (100) einen Impuls-Funk-Ultrabreitband(IR-UWB)-Kommunikation-Sensor aufweist,
die Sensor-Einheit (100) biometrische Informationen der Person (5) detektiert mittels Sendens eines Signals und Empfangens des von der Person (5) reflektierten Signals, und
die biometrischen Informationen zumindest eine von Herzrate, Bewegung oder Atmung der Person aufweisen.

2. System (10) gemäß Anspruch 1, wobei die Ermittlungseinheit (200) eine Empfindlichkeit der Sensor-Einheit (100) gemäß einer Ansteuerungsstärke der Innenraum-Steuervorrichtung (20) einstellt.

3. System (10) gemäß Anspruch 1 oder 2, wobei die Innenraum-Steuervorrichtung (20) eine Klimaanlage (21) ist, bei welcher eine Luftströmung in dem Innenraum stark wird, wenn eine Ansteuerungsstärke der Klimaanlage (21) stark wird, und die Ermittlungseinheit (200) eine Empfindlichkeit der Sensor-Einheit (100) reduziert, um einen Detektierungsfehler der Sensor-Einheit aufgrund der starken Änderung der Luftströmung zu verhindern.

4. System (10) gemäß irgendeinem der Ansprüche 1 bis 3, wobei die Ermittlungseinheit (200) aufweist:
eine Analyseeinheit (210), welche analysiert, ob eine in dem Innenraum detektierte Bewegung von der Person (5) erzeugt wird oder nicht, auf der Basis der mittels der Sensor-Einheit (100) detektierten Informationen, und
eine Empfindlichkeit-Einstellung-Einheit (230), welche eine Empfindlichkeit der Sensor-Einheit (100) steuert, auf der Basis einer Ansteuerungsstärke der Innenraum-Steuervorrichtung (20).

5. System (10) gemäß irgendeinem der Ansprüche 1 bis 4, welches ferner aufweist:
eine Big-Data-Erzeugungseinheit (400), welche Informationen über ein Verhaltensmuster der Person (5), welche sich in dem Innenraum befindet, ein Ansteuerungsmuster der Innenraum-Steuervorrichtung (20) und den Innenraum speichert, und dadurch Informationen über eine spezifische Person (5) sammelt.

6. System (10) gemäß Anspruch 5, wobei die von der Big-Data-Erzeugungseinheit (400) erzeugten Informationen über die spezifische Person (5) mit einem Reservierungssystem (50) einer von der spezifischen Person (5) bewohnten Unterkunft assoziiert werden, und
die in der Big-Data-Erzeugungseinheit (400) gespeicherten Informationen zum Reservierungssystem (50) der Unterkunft übertragen werden, wenn die spezifische Person (5) die Unterkunft reserviert.

7. System (10) gemäß irgendeinem der Ansprüche 1 bis 6, wobei die Ermittlungseinheit (200) die Anzahl an Personen (5) ermittelt, welche sich in dem Innenraum befinden, und
die Steuereinheit eine Stärke der Innenraum-Steuervorrichtung (20) auf der Basis der Anzahl an Personen (5) steuert.

8. System (10) gemäß irgendeinem von Anspruch 1, wobei die Ermittlungseinheit (200) die ermittelten Informationen zu einem Endgerät (500) übertragt, welches außerhalb des Innenraums angeordnet ist.

9. System (10) gemäß irgendeinem der Ansprüche 1 bis 8, wobei die Sensor-Einheit (100) mehrfach in dem Innenraum bereitgestellt ist,
die mehreren Sensor-Einheiten (100) detektieren, ob die Person (5) in einem privaten Raum oder einem öffentlichen Raum von den Innenräumen anwesend ist oder nicht, und
die Innenraum-Steuervorrichtung (20) in jedem des privaten Raums und des öffentlichen Raums bereitgestellt ist.

10. System gemäß Anspruch 9, wobei die Ermittlungseinheit (200) ermittelt, ob die Person (5) in dem privaten Raum und dem öffentlichen Raum anwesend ist oder nicht, auf der Basis der mittels der mehreren Sensor-Einheiten (100) detektierten Informationen, und
die Steuereinheit (300) die Innenraum-Steuervorrichtung (20) steuert, welche in einem Raum angeordnet ist, in welchem keine Person (5) anwesend ist, von dem privaten Raum und dem öffentlichen Raum.

11. System (10) gemäß Anspruch 9 oder 10, wobei die Innenraum-Steuervorrichtung (20) eine Klimaanlage (21) und eine Beleuchtungseinheit (23) aufweist, und
die Steuereinheit (300) eine Ansteuerungsstärke der Klimaanlage (21), welche in dem Raum angeordnet ist, in welchem die Person (5) nicht anwesend ist, von dem privaten Raum und dem öffentlichen Raum, steuert, um niedriger als eine gegenwärtige Ansteuerungsstärke zu sein, und eine Beleuchtungsintensität der Beleuchtungseinheit (23) steuert, um niedriger als eine gegenwärtige Intensität zu sein.

12. System (10) gemäß Anspruch 9 oder 10, wobei die Innenraum-Steuervorrichtung (20) eine Klimaanlage (21) und eine Beleuchtungseinheit (23) aufweist, und,
wenn sich eine Person (5), die sich in einem beliebigen Raum des privaten Raums und des öffentlichen Raums befindet, in den anderen Raum bewegt, die Steuereinheit (300) eine Ansteuerungsstärke der Klimaanlage (21) steuert, um niedriger als eine gegenwärtige Stärke zu sein, und eine Beleuchtungsintensität der Beleuchtungseinheit (23) steuert, um niedriger als eine gegenwärtige Intensität zu sein, in der Klimaanlage (21) und der Beleuchtungseinheit (23), welche in dem einen beliebigen Raum angeordnet sind.

13. System (10) gemäß Anspruch 12, wobei, wenn sich eine Person (5), welche sich in einem beliebigen Raum des privaten Raums und des öffentlichen Raums befindet, zu dem anderen Raum bewegt, die Steuereinheit (300) die Ansteuerungsstärke der Klimaanlage (21) steuert, um höher als die gegenwärtige Stärke zu sein, und die Beleuchtungsintensität der Beleuchtungseinheit (23) steuert, um höher als die gegenwärtige Intensität zu sein, in der Klimaanlage (21) und der Beleuchtungseinheit (23), welche in dem anderen Raum angeordnet sind.

## Revendications

1. Système de bâtiment intelligent (10), comprenant :
une unité de détection (100) disposée dans un espace intérieur pour détecter des informations qui comprennent la présence d'une personne (5) située dans l'espace intérieur et des informations biométriques de la personne (5) située dans l'espace intérieur ;
une unité de détermination (200) qui détermine si la personne (5) se situe ou non dans l'espace intérieur sur la base des informations acquises par l'unité de détection (100) ;
une unité de commande (300) qui commande un contrôleur intérieur (20) disposé à l'intérieur de l'espace intérieur sur la base des informations déterminées par l'unité de détermination (200) ; et
une unité de stockage (250),
**caractérisé en ce que**
l'unité de stockage (250) stocke des informations qui concernent des déplacements répétitifs qui se produisent dans l'espace intérieur,
où l'unité de détermination (200) détermine que des informations identiques aux informations concernant les déplacements répétitifs stockées dans l'unité de stockage (250) parmi les informations détectées par l'unité de détection (100) ne sont pas des informations biométrique de la personne (5) permettant de déterminer si la personne (5) se situe ou non dans l'espace intérieur sur la base des informations biométriques de la personne (5),
les mouvements répétitifs signifient un déplacement avec un cycle constant,
l'unité de détection (100) inclut un capteur de communication à bande ultralarge à impulsions radio (IR-UWB),
l'unité de détection (100) détecte les informations biométriques de la personne (5) en transmettant un signal et en recevant le signal réfléchi par la personne (5), et
les informations biométriques incluent l'un au moins d'une fréquence cardiaque, d'un déplacement, ou de la respiration de la personne.

2. Système (10) selon la revendication 1, où l'unité de détermination (200) ajuste la sensibilité de l'unité de détection (100) selon une force de commande du contrôleur intérieur (20).

3. Système (10) selon la revendication 1 ou 2, où le contrôleur intérieur (20) est un climatiseur (21), dans lequel un flux d'air devient fort dans l'espace intérieur lorsque la force de commande du climatiseur (21) devient forte, et l'unité de détermination (200) réduit la sensibilité de l'unité de détection (100) afin d'empêcher une erreur de détection de l'unité de détection, due au changement fort du flux d'air.

4. Système (10) selon l'une quelconque des revendications 1 à 3, où l'unité de détermination (200) inclut :
une unité d'analyse (210) qui analyse si un déplacement détecté dans l'espace intérieur est généré ou non par la personne (5) sur la base des informations détectées par l'unité de détection (100) ; et
une unité d'ajustement de la sensibilité (230) qui commande la sensibilité de l'unité de détection (100) sur la base d'une force de commande du contrôleur intérieur (20).

5. Système (10) selon l'une quelconque des revendications 1 à 4, comprenant en outre :
une unité de génération de mégadonnées (400) qui stocke des informations sur un modèle de comportement de la personne (5) située dans l'espace intérieur, un modèle de commande du contrôleur intérieur (20), et l'espace intérieur, en collectant de ce fait des informations sur une personne spécifique (5).

6. Système (10) selon la revendication 5, où les informations sur la personne spécifique (5) générées par l'unité de génération de mégadonnées (400) sont associées à un système de réservation (50) d'un logement qui est occupé par la personne spécifique (5), et
les informations stockées dans l'unité de génération de mégadonnées (400) sont transmises au système de réservation (50) du logement lorsque la personne spécifique (5) réserve le logement.

7. Système (10) selon l'une quelconque des revendications 1 à 6, où l'unité de détermination (200) détermine le nombre de personnes (5) qui se situent dans l'espace intérieur, et
l'unité de commande commande la force du contrôleur intérieur (20) sur la base du nombre de personnes (5).

8. Système (10) selon la revendication 1, où l'unité de détermination (200) transmet les informations déterminées à un terminal (500) disposé à l'extérieur de l'espace intérieur.

9. Système (10) selon l'une quelconque des revendications 1 à 8, où l'unité de détection (100) est disposée en plusieurs exemplaires dans l'espace intérieur,
les multiples unités de détection (100) détectent si la personne (5) est présente ou non dans un espace privé ou dans un espace public parmi les espaces intérieurs, et
le contrôleur intérieur (20) est disposé dans chaque espace privé et dans chaque espace public.

10. Système selon la revendication 9, où l'unité de détermination (200) détermine si la personne (5) est présente ou non dans l'espace privé et dans l'espace public sur la base des informations détectées par les multiples unités de détection (100), et
l'unité de commande (300) commande le contrôleur intérieur (20) disposé dans un espace où aucune personne (5) n'est présente, parmi l'espace privé et l'espace public.

11. Système (10) selon la revendication 9 ou 10, où le contrôleur intérieur (20) inclut un climatiseur (21) et une unité d'éclairage (23), et
l'unité de commande (300) commande qu'une force de commande du climatiseur (21) disposé dans l'espace où la personne (5) n'est pas présente parmi l'espace privé et l'espace public soit inférieure à la force de commande actuelle et qu'une intensité d'éclairage de l'unité d'éclairage (23) soit inférieure à l'intensité actuelle.

12. Système (10) selon la revendication 9 ou 10, où le contrôleur intérieur (20) inclut un climatiseur (21) et une unité d'éclairage (23), et
quand une personne (5) qui se situe dans un espace quelconque de l'espace privé et de l'espace public se déplace vers l'autre espace, l'unité de commande (300) commande qu'une force de commande du climatiseur (21) soit inférieure à une force actuelle et qu'une intensité de l'éclairage de l'unité d'éclairage (23) soit inférieure à une intensité actuelle dans le climatiseur (21) et dans l'unité d'éclairage (23) disposés dans ledit un espace quelconque.

13. Système (10) selon la revendication 12, où quand une personne (5) qui se situe dans un espace quelconque de l'espace privé et de l'espace public se déplace vers l'autre espace, l'unité de commande (300) commande que la force de commande du climatiseur (21) soit supérieure à la force actuelle, et que l'intensité de l'éclairage de l'unité d'éclairage (23) soit supérieure à l'intensité actuelle dans le climatiseur (21) et dans l'unité d'éclairage (23) disposés dans l'autre espace.
